# EUROPÄISCHE PATENTSCHRIFT

(11) **EP 2 777 629 B1**
(45) Veröffentlichungstag und Bekanntmachung des Hinweises auf die Patenterteilung: **27.07.2016**
(21) Anmeldenummer: 14158937.4
(22) Anmeldetag: 11.03.2014
(51) Int. Cl.: A61F 2/44

(54) **Aufspreizbares Implantat für die Wirbelsäule**
Spreadable implant for the spinal column.
Implant expansible pour la colonne vertébrale.

(30) Priorität: 12.03.2013 DE 102013102451
(43) Veröffentlichungstag der Anmeldung: 17.09.2014
(73) Patentinhaber: Böhm, Heinrich, Dr., 99425 Weimar (DE)
(72) Erfinder: Böhm, Heinrich, 99425 Weimar (DE); Burger, Andreas, 78532 Tuttlingen (DE); Widmaier, Gerd, 78532 Tuttlingen (DE); Wenzler, Klaus, 78665 Frittlingen (DE)
(74) Vertreter: Mammel und Maser

(56) Entgegenhaltungen:
- WO-A1-2013/158294
- WO-A2-2009/064787
- WO-A2-2011/011609
- US-A1- 2011 172 716
- US-A1- 2013 053 966

## Beschreibung

Die vorliegende Erfindung betrifft ein aufspreizbares implantat, insbesondere betrifft die Erfindung ein spreizbares höhenadaptierbares Implantat für die Lenden- und Brustwirbelsäule, das als Ersatz für Wirbelkörper und/oder Bandscheibenräume bei Versteifungsoperationen dienen kann.

Solche lumbalen und thoracalen implantate werden zur interkorporalen Fusion (Versteifung der Wirbelkörper) eingesetzt. Prinzipiell ist jedoch auch eine intrakorporale Anwendung möglich.

In der Konfiguration als Wirbelkörperersatz kann das Implantat bei Zerstörungen oder Formdefekten jeder Art eingesetzt werden: so bei Tumoren, Wirbelbrüchen, Spätfolgen nach Wirbelbrüchen sowie Infektionen oder angeborenen Fehlbildungen eines oder mehrerer Wirbelkörper.

In der Konfiguration als Zwischenwirbelersatz, "intersomatischer Cage" = "aufspreizbarer Cage", kann das Implantat zur Wiederherstellung der vorderen Säule der Wirbelreihe im Rahmen von Versteifungseingriffen verwendet werden. Hier sind Anwendungsgebiete die degenerativen Wirbelsäulenerkrankungen, Segmentfehlstellungen und Segmentinstabilitäten verschiedenster Ursachen.

Im Allgemeinen wird zunächst der Zwischenwirbelraum vom Rücken beziehungsweise vom Brust- oder Bauchraum her ausgeräumt, bei Wirbelkörperersatz der Wirbelkörper entfernt und dann das Implantat in den entstandenen Zwischenraum eingeführt, um zur Stellungskorrektur sowie als Abstandshalter zur mechanisch stabilen Verbindung zwischen den angrenzenden Wirbelsäulenabschnitten zu dienen.

Es hat sich als vorteilhaft erwiesen, solche Implantate als "Cage", das heißt in Form eines Käfigs, auszubilden, damit in dessen Innenraum beispielsweise Knochenmaterial oder Knochenersatzmaterialien zur Beschleunigung des Einheilens eingebracht werden können. Hierdurch kann die Fusion des Cages ermöglicht bzw. beschleunigt werden.

Über einen Spreizmechanismus nach dem Einbringen in den Wirbelsäulendefekt aufspreizbare Cages haben den Vorteil, technisch einfacher und für die Ankerwirbel schonender implantiert werden zu können: durch das stufenlose Aufspreizen wird das Implantat einerseits fest verklemmt und fixiert. Andererseits erlaubt es individuelle Stellungskorrekturen der angrenzenden Wirbel, um normale geometrische Verhältnisse durch Schaffung von Lordose der Lendenwirbelsäule oder Korrektur einer vermehrten Brustkyphose wieder herzustellen.

Mit Ausnahme des Einsatzes bei bösartigen Tumoren soll das Durchwachsen des Implantats bzw. des rekonstruierten Wirbelsäulenabschnitts mit körpereigenem Knochengewebe ermöglicht werden. Deshalb weisen solche Implantate vorzugsweise Durchbrüche auf.

Abhängig vom Design gewährleisten Cages neben mechanischer Stabilität des Korporektomiedefektes bzw. struktureller Integrität des Zwischenwirbelraumes während des knöchernen Durchbauungsprozesses auch den Schutz des Rückenmarks vor sich verschiebenden Knochenfragmenten.

Ein generelles Problem bei den bekannten Implantaten besteht darin, dass die Implantate nach dem Einbau in die Nachbarwirbel einsinken können, wodurch die Lagebeziehung der Wirbel zueinander und die Gesamtstatik gestört werden. Dies kann insbesondere bei vermindertem Kalksalzgehalt - Osteoporose - der Wirbelkörper auftreten.

Es sind zylinderförmige Implantate bekannt, die an der Zylinderoberfläche ein Schraubgewinde aufweisen. Diese Implantate werden horizontal in den Zwischenwirbelraum eingeschraubt, nachdem zuvor Boden- und Deckplatte der Wirbelkörper mit Bohrinstrumenten arrodiert, das heißt partiell entfernt wurden. Nachteilig an diesen zylinderförmigen Implantaten ist, dass diese eine relativ kleine Auflagezone haben und ein vergleichsweise starkes Einsinken in die Wirbelkörper zeigen und verkanten.

Aus der EP 1 415 622 A1 sind Spreizimplantate mit einer eher quaderförmigen Grundform bekannt, die zwei an einem Ende miteinander verbundene Schenkel aufweisen, wobei die Schenkel mit einem zwischen den Schenkeln angeordneten Schieber aufspreizbar sind, der die Schenkel im aufgespreizten Zustand über ihre gesamte horizontale Breite abstützt und in der Endposition einrastet. Obwohl sich diese Implantate durch eine größere Auflagefläche auf der Endplatte des Nachbarwirbels auszeichnen, ist die Art der Aufspreizung designbedingt sehr begrenzt und immer winkelförmig vorgegeben. Wegen der dann wiederum häufig unvermeidbaren punktförmigen Belastung sinken auch sie oft deutlich in die Nachbarwirbel ein und verlieren so ihre Aufspreiz- bzw. Korrekturfunktion. Darüber hinaus ist designbedingt eine Implantation solcher Cages von der Seite der Wirbelsäule nicht möglich und auf strikt dorsale - selten zusätzlich strikt von vorn kommende - Implantationsrichtung beschränkt. Der Einsatz als Wirbelkörperersatz ist bei beiden vorgenannten Modellen unmöglich.

Aus der US 2012/017955 A1 ist ein spreizbares Implantat bekannt, das vier Getriebe umfasst, von denen jeweils zwei durch Zahnräder miteinander gekoppelt sind. Ober- und unterhalb der Zahnräder weisen die Getriebe jeweils eine Gewindespindel mit unterschiedlicher Drehrichtung auf, so dass sich bei Betätigung des Zahnrads gleichzeitig das obere und das untere Gewinde aus der oberen und unteren Platte herausschraubt und damit die obere gegenüber der unteren Platte gespreizt wird bzw. bei einem gleichzeitigen Hineinschrauben der Gewinde in die Platte das Implantat komprimiert wird. Das Implantat ist konstruktiv aufwändig und weist bei großem Bauvolumen nur einen geringen Hub auf. Eine knöcherne Durchbauung ermöglicht dieses Implantat nicht. Ein weiterer Nachteil ist die Zylinderform des Implantats mit den kreisförmigen Platten, die zur Verankerung an/in den Wirbelstützflächen dienen. Bei der Positionierung solcher Implantate kommt das Implantat gerade in der Mitte der Wirbelstützfläche zu liegen, somit dem am wenigsten harten Bereich der gesamten Wirbelstützfläche, so dass das Implantat einsinkt. Aufgrund der Zylinderform und des Angriffs des Bedieninstruments in der Zylindermitte kann das Implantat auch nicht mit dem Bedieninstrument bei einer dorsalen Implantation an den Nervenwurzeln vorbei in die richtige Position geschwenkt werden. Für eine dorsale, dorsolaterale oder TLIF-Implantation ist das Implantat somit nicht geeignet.

Aus der US 5,236,460 sind spreizbare teleskopartige Implantate bekannt. Der äußere Implantatkörper, der rohrförmig ist, weist eine axiale Bohrung auf, in der der innere Implantatkörper gleitend geführt ist. Am oberen und unteren Ende der Implantatkörper sind Platten mit Dornen zur Verankerung vorgesehen. Die Spreizung des Implantats erfolgt hydraulisch über eine Flüssigkeit oder ein härtbares Harz. Hierzu weist der äußere Implantatkörper eine Öffnung zum Eintritt des Fluids auf, das dann in eine Kavität im unteren Ende des inneren Implantatkörpers gelangt und diesen axial gleitend nach oben schiebt. Das Implantat wird über eine spezielle Klammervorrichtung, die auf die obere Plattform aufgeschraubt wird, mit den Wirbelkörpern verschraubt.

Die US 2011/0160861 A1 lehrt ein spreizbares Implantat mit einer eher rechteckigen Grundform mit zwei Getrieben. Die Getriebe sind über eine Antriebsstange, die den Boden in Längsrichtung durchquert, gekoppelt. Das Einbringinstrument wird in Längsrichtung an den kurzen Seiten des Implantats angeschraubt, so dass das Implantat insbesondere für eine seitliche, d.h. laterale oder XLIF-Implantation eingesetzt werden kann. Für die TLIF-Technik (Transformaminal/Transarticular Lumbar Interbody Fusion) ist dieses Implantat nicht geeignet, da es nicht um die Nervenwurzeln herum in die optimale Position gebracht werden kann. Bei Anwendung der PLIF-Technik muss bei diesem Implantat über zwei Zugänge implantiert werden. In Bezug auf die ALIF-Technik, d.h. der vorderen Implantation durch den Bauch, kann dieses Implantat nur von der Seite und somit nicht in die optimale Position gebracht werden. Ein weiterer Nachteil ist, dass das Implantat kein Durchwachsen des Knochens ermöglicht.

Aus der WO 2009/064787 sind spreizbare Implantate mit einem doppelten teleskopartigen Verstellmechanismus bekannt, die eine obere und eine untere Platte zur Verankerung an den Wirbelstützflächen aufweisen. Der Verstellmechanismus ist über Zahnräder gekoppelt, d.h. eine Rotationsbewegung wird von dem einen auf das andere Zahnrad und somit von dem einen auf den anderen Verstellmechanismus übertragen. Der Verstellmechanismus umfasst jeweils ein mit der oberen und der unteren Platte fest verbundenes Gewinde und zwei mittlere Antriebe, mittels derer das Implantat gespreizt werden kann. Die Betätigung soll gemäß dieser Druckschrift durch einen Stab mit Kegelradgetriebe in nicht näher erläuterter Weise erfolgen.

Dieses Implantat ist als Bandscheibenersatz geeignet. Infolge der größeren Fläche der oberen und unteren Platte wird die Gefahr einer sekundären Stellungsveränderung und des Einsinkens minimiert. Der mit diesem Verstellmechanismus maximal erzielbare Hub ist allerdings vergleichsweise gering. Nachteilig ist auch, dass diese Implantate bislang nicht auf einfache Weise in minimal invasiver Technik eingebracht, aufgespreizt und fixiert werden können, dass der Aufbau des Implantats aufwändig konstruiert ist und zudem keine knöcherne Durchbauung erlaubt.

Aus der US 2011/0172716 ist ein expandierbares Implantat bekannt, das eine obere und eine untere Platte, die zur Verankerung in den Wirbelstützflächen dienen und zwei Getriebe, die miteinander über Zahnräder gekoppelt sind, aufweist. Die Getriebe können mit dem Finger oder einem Werkzeug angetrieben werden.

Die Aufgabe der Erfindung besteht darin, ein im Körper aufspreizbares und dadurch korrigierendes Implantat zur Versteifung des Bandscheibenraumes oder nach Entfernung von Wirbelkörpern anzugeben, das auf einfach handhabbare und bedienbare und zudem sichere Weise auch in minimal invasiver Technik implantierbar ist und bei dem sichergestellt ist, dass keine Knochenfragmente in den Rückenmarkskanal geraten und dort das Rückenmark einengen oder schädigen können.

Diese Aufgabe wird durch ein Implantat mit den Merkmalen des Anspruchs 1 und ein Bedieninstrument mit den Merkmalen des Anspruchs 11 gelöst.

Das erfindungsgemäße Implantat, das eine obere und eine untere Platte, die zur Verankerung in den Wirbelstützflächen dienen, aufweist und wenigstens zwei Getriebe zum Aufspreizen des Implantats, die miteinander gekoppelt sind, umfasst weiterhin eine Antriebswelle an dem einen Getriebe, mittels der eine auf die Antriebswelle eines Bedieninstruments wirkende Kraft auf das Getriebe übertragen werden kann. Weiterhin umfasst das spreizbare Implantat Mittel zur Befestigung, Verschraubung oder starren Fixierung des Bedieninstruments.

Damit kann das Implantat an dem Bedieninstrument sowohl starr fixiert werden als auch gleichzeitig mit demselben Bedieninstrument das Getriebe angetrieben und damit über die Kopplung der beiden Getriebe das Implantat aufgespreizt werden.

Dies ermöglichst eine einfache Handhabung des Implantats während der Operation, da nur ein Bedieninstrument erforderlich ist, das sowohl die Fixierung an dem Implantat als auch die Aufspreizung als auch die anschließend noch näher beschriebene Fixierung der Spreizstellung ermöglicht.

Vorzugsweise ist die Antriebswelle, die starr mit der Gewindehülse verbunden ist, ein Tellerrad bzw. ein Kegelring, auf die mit einem später beschriebenen Kegelantrieb des Bedieninstruments eine Drehbewegung übertragen werden kann.

Das sich an der äußeren Mantelfläche der Gewindehülse radial nach außen erstreckende Tellerrad ist von außen gut für den Kegelantrieb des Bedieninstruments zugänglich. Infolge der nachfolgend noch beschriebenen starren Befestigung des Bedieninstruments an dem Implantat ist zudem die Umgebung des Kegelantriebs abgeschirmt, so dass bei Betätigung des Bedieninstruments ein Einquetschen des Gewebes und eine Beeinträchtigung der Funktion des Kegelradgetriebes vermieden werden.

Für die einfache Bedienung und Implantation ist es zudem erforderlich, dass das Bedieninstrument starr an dem Implantat befestigt werden kann. Hierzu weist das Implantat erfindungsgemäß Mittel zur Befestigung, Verschraubung oder sonstigen starren Fixierungen des Bedieninstruments auf. Die Verbindung zwischen Implantat und Bedieninstrument muss selbstverständlich lösbar sein. Vorzugsweise umfasst das Bedieninstrument ein Schraubgewinde (Außengewinde), das in einer entsprechenden Bohrung im Implantat verschraubt werden und das nach der erfolgten Positionierung und Spreizung wieder entfernt werden kann. Die Verbindung kann jedoch auch anderweitig erfolgen.

Da das Bedieninstrument sowohl mit der Antriebswelle (Kegelantrieb) an der Antriebswelle (Tellerrad) der Gewindehülse angreift, als auch die Festziehwelle des Bedieninstruments mit der Bohrung des Implantats verschraubt wird und der Abstand zwischen Schraubgewinde der Festziehwelle und dem Kegelantrieb in dem Bedieninstrument konstant ist, darf auch der Abstand zwischen der Bohrung zur Aufnahme des Schraubgewindes in dem Implantat und dem Tellerrad nicht veränderbar sein, d.h. dass die Bohrungen zur Aufnahme der Schraubgewinde in dem Implantat starr mit der Platte verbunden sein müssen, in der die Gewindehülse mit dem Tellerrad drehbar gelagert ist.

Durch den doppelten Verstellmechanismus wird das Spreizen durch zwei Getriebe eingeleitet und so ein Verkanten des Getriebes vermieden. Ein weiterer wesentlicher Vorteil des doppelten Verstellmechanismus ist, dass infolge der doppelten Aufspreizbewegung auch die Fläche der oberen und unteren Platte vergrößert und damit der auf die Wirbelkörper pro Flächenelement wirkende Druck vermindert werden kann. Die Verringerung des Flächendrucks verringert zudem das unerwünschte Einsinken des Implantats. Selbstverständlich kann der Verstellmechanismus auch mehr als zwei Getriebe, zum Beispiel drei oder vier, aufweisen.

Durch die Ausbildung der Getriebeelemente mit einer Gewindehülse und einer Gewindespindel und Kopplung derselben wird die Bedienung erleichtert und eine gleichmäßige Spreizbewegung erreicht.

Durch das Zusammenwirken von Gewindehülse und Gewindespindel lässt sich der erforderliche Abstand zwischen der oberen und unteren Platte in der gewünschten Spreizung präzise und auf einfache Weise einstellen.

Die Kopplung erfolgt über Zahnräder, die mit den nach außen weisenden Mantelflächen der Gewindehülsen starr verbunden sind, wobei jeweils zwei Zahnräder auf unterschiedlichen Gewindehülsen ein Zahnradpaar bilden.

Selbstverständlich korrespondieren die Antriebswelle des Bedieninstruments und die Befestigungsmittel des Bedieninstruments zu den korrespondierenden Wellen/Befestigungsmitteln des Implantats. Vorzugsweise wird das Bedieninstrument in Bohrungen, insbesondere Gewindebohrungen, des Implantats eingeschraubt, und zwar mit einem Außengewinde am proximalen Ende einer Festziehwelle.

Die Antriebswelle des Implantats ist vorzugsweise als Kegelantrieb ausgestaltet, der sich am proximalen Ende einer Drehwelle befindet. Drehwelle und Festziehwelle sind parallel zueinander angeordnet.

Vorzugsweise weist das Bedieninstrument eine Halterung aus Rohren und Aufnahmen auf, wobei die Rohre zur Aufnahme der Festziehwellen und/oder eines Schraubinstruments dienen und auch die Drehwelle in der Halterung geführt wird.

Mit dem erfindungsgemäßen Implantat nebst Bedieninstrument wird erstmals ein Implantat bereitgestellt, das universell einsetzbar ist, da es sowohl für die Implantation in Rücken-, Seiten- und Bauchlage in der XLIF-, ALIF-, TLIF-Technik verwendet werden kann, und zwar auch bei der Implantation des 4. bzw. 5. Lendenwirbels. Dies wird dadurch erreicht, dass das Implantat mehrere unterschiedlich orientierte Teilabschnitte (d.h. die Flächen der Teilabschnitte sind nicht koplanar zueinander) aufweist, an denen das Bedieninstrument befestigt werden kann. Je nachdem, an welchem Teilabschnitt das Bedieninstrument befestigt ist, kann ein unterschiedlicher Winkel zwischen der Längsachse des Implantats und der Längsachse des Bedieninstruments und damit eine andere Orientierung der Längsachse des Implantats zu der Längsachse des Bedieninstruments erhalten werden.

Nach Einbringung in den Zwischenwirbeldefekt oder Korporektomiedefekt erfolgt die Aufspreizung bis zum Erreichen der mechanisch stabilen Verankerung und ggf. Stellungskorrektur. Die erreichte Spreizung ist während der Operation jederzeit veränderbar, um eine etwaige Umpositionierung vorzunehmen oder eine Überspreizung des Wirbelsäulenabschnitts durch Betätigung des Kegelantriebs am Bedieninstrument zurückzufahren. Ohne Betätigung des Kegelantriebs verbleibt das Implantat jedoch infolge der Reibung sicher in der eingestellten gespreizten Stellung und kann nach Erreichen der gewünschten Spreizung danach durch einen Verschlussstift gesichert werden.

Infolge der Stabilität des Implantats durch die doppelten Säulen ist es möglich, die Fläche, die Winkelgrade und auch die Oberflächenkonfiguration der oberen und unteren Platten relativ frei zu wählen und damit den Formschluss zu optimieren und dem unerwünschten Einsinken der Implantate entgegen zu wirken.

Das erfindungsgemäße Implantat ist vorzugsweise aus Reintitan oder einer Titanlegierung wie Ti₆Al₄, Tantal, Nitinol, einem Kunststoffmaterial wie Polyetheretherketon (PEEK) oder anderen Materialien, die als Implantatwerkstoffe geeignet sind.

Das erfindungsgemäße Implantat wird wie folgt implantiert:

In Bauch-, Seiten- oder Rückenlage wird über einen dorsalen oder ventralen abdominalen oder thorakalen Zugang der Zwischenwirbelraum freigelegt, dann vollständig ausgeräumt. Soll ein Wirbelkörper entfernt werden, wird dies an der zweiten angrenzenden Bandscheibe wiederholt und anschließend die Korporektomie durchgeführt. Entsprechend der Indikation können dabei entweder nur Teile des Wirbelkörpers reseziert werden oder alle Anteile des Wirbeikörpers inklusive von Hinterkante bzw. Knochenfragmenten aus dem Spinalkanal entfernt werden. Dann werden die Endplatten der Ankerwirbel entknorpelt und angeraut, um das Knochenwachstum anzuregen.

Anschließend wird das größen- und formadaptiert ausgewählte Implantat mittels des erfindungsgemäßen Bedienelements, einem kombinierten Halte-, Aufspreiz- und Arretierungsinstrument, in den ausgeräumten Defekt eingebracht und aufgespreizt. Die Geometrie des Implantats und des Bedieninstruments erlaubt dabei sowohl die Einbringung von vorn, von beiden Seiten als auch von hinten seitlich des Rückenmarkssackes. Der operative Zugang kann konventionell offen, aber auch in minimal invasiver Technik erfolgen. Insbesondere bei der Schlüssellochtechnik erweist sich der Vorteil der symmetrischen Einleitung relativ starker Korrekturkräfte.

Da die Endplattengröße und -konfiguration relativ frei mit den zwei Extensionskörpern kombinierbar ist, kann eine große Abstützfläche bei Osteoporose ebenso verwendet werden, wie eine kleine bei solchen Wirbelfrakturen, bei denen Teile des Wirbelkörpers erhalten werden können.

Eine optional an den Innenzylinder angebrachte Kante führt während der Aufspreizung zu einer Anfrischung der nicht zur Abstützung erforderlichen Ankerwirbelendplatte und erlaubt so, dass die jeweils schwerer zugängliche Endplatte nicht in einem eigenen Arbeitsgang angefrischt werden muss.

Anschließend erfolgt die Stellungskontrolle mittels intraoperativer Bildgebung, meist mittels Durchleuchtung. Liegt eine suboptimale Implantatlage vor, wird durch Drehen des Aufspreizgeräts in Gegenrichtung das Implantat zusammengefahren, umpositioniert und erneut aufgespreizt.

Das Implantat kann mit Knochen (z.B. Spongiosa) oder anderen Materialien (Calciumphosphatpräparate) befüllt werden. Die Rekonstruktion der vorderen Säule ist damit beendet. Die Kombination mit einer dorsalen Stabilisierung wird vor allem im thorako-lumbalen und lumbalen Bereich dringend empfohlen. Je nach Knochenqualität und Blutverlust kann die Mobilisation des Patienten dann am gleichen Tage erfolgen.

Die Erfindung wird nachfolgend anhand eines Ausführungsbeispiels näher beschrieben.

Es zeigen:

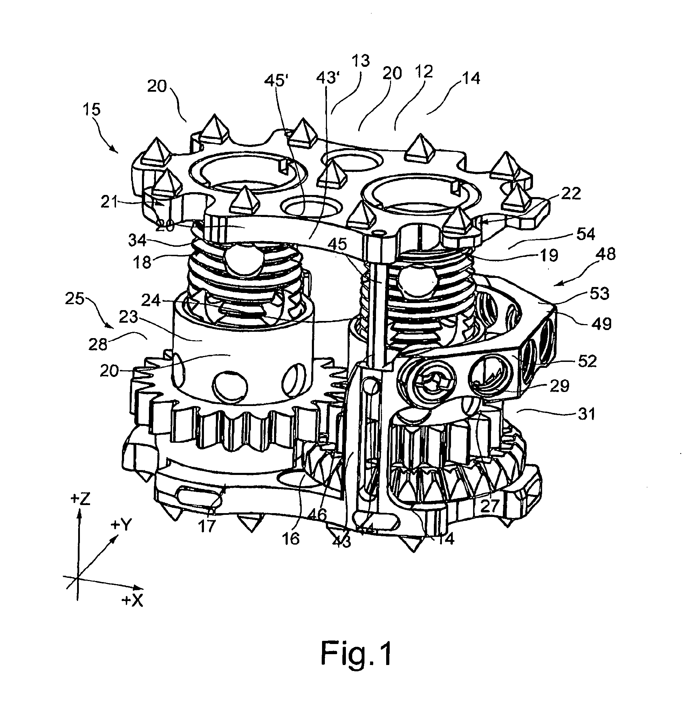

Figur 1 eine perspektivische Ansicht des erfindungsgemäßen Implantats in maximaler Spreizung,
Figur 2 eine Draufsicht auf das Implantat aus Figur 1 von oben,

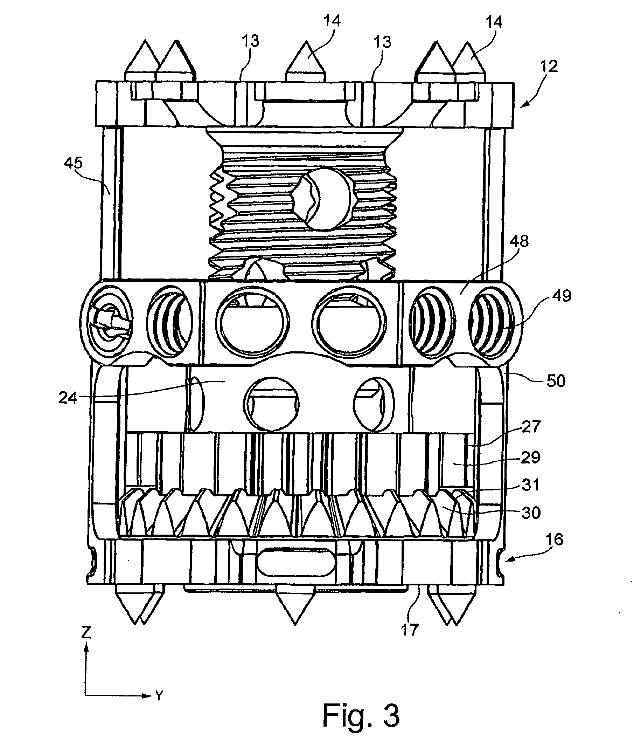

Figur 3 eine Seitenansicht auf das Implantat aus Figur 1 in -x-Richtung,

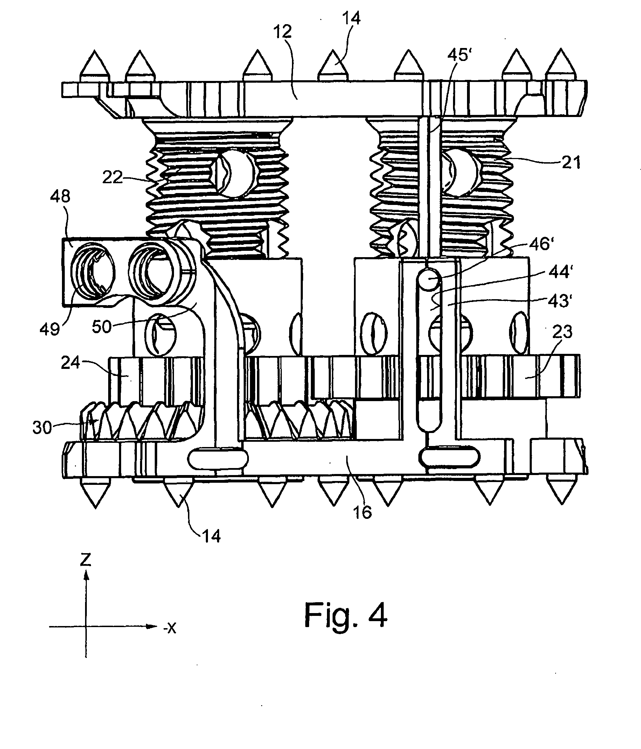

Figur 4 eine Seitenansicht (xz-Richtung) auf das Implantat aus Figur 1 mit Blickrichtung von hinten (-γ-Richtung),

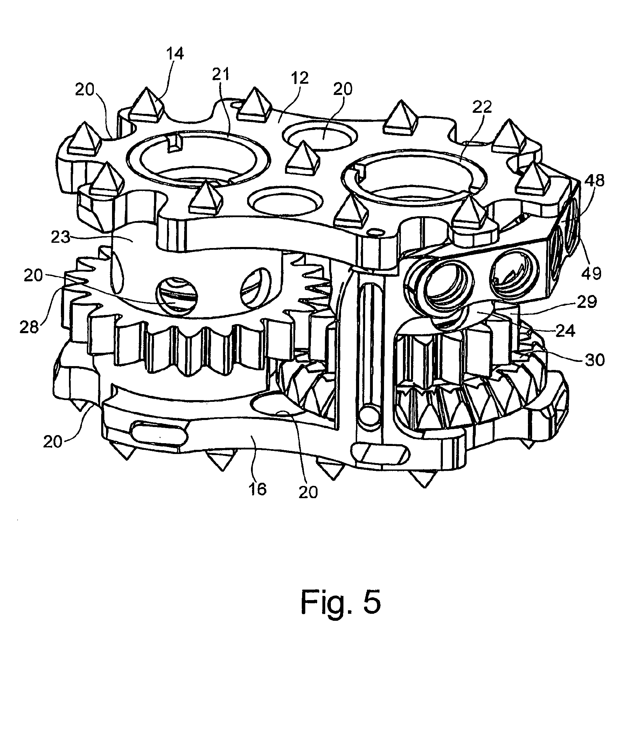

Figur 5 das Implantat aus Figur 1, jedoch im ungespreizten Zustand,
Figur 6 das erfindungsgemäße Bedieninstrument und das Schraubinstrument 80 in perspektivischer Ansicht,
Figur 7 die Seitenansicht des proximalen Endes des Instruments aus Figur 6,
Figur 8 die perspektivische Ansicht auf das proximale Ende des Bedieninstruments aus Figur 6 und 7, und

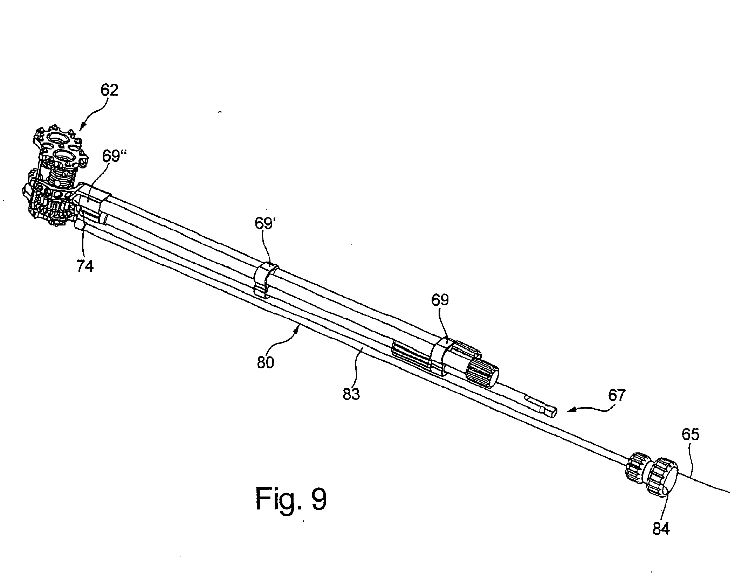

Figur 9 das Implantat im aufgespreizten Zustand mit daran befestigtem Bedieninstrument
Figur 10 die Draufsicht auf eine andere Variante des Horizontalabschnitts 48 mit vier verschiedenen Teilabschnitten 52, 53, 54 und 55, an denen das Bedieninstrument unter verschiedenen Winkeln mit dem Implantat verschraubt werden kann.

Das Implantat umfasst eine obere Platte 12 und eine untere Platte 16, an deren Außenseiten 13, 17 Dorne 14 vorgesehen sind, die zur Verankerung an/in den Wirbelstützflächen dienen.

Zwischen der oberen Platte 12 und der unteren Platte 16 befinden sich zwei Getriebe, durch die die obere Platte 12 gegenüber der unteren Platte 16 verschiebbar ist, d.h. das Implantat gespreizt werden kann.

Die Getriebe bestehen jeweils aus einer Gewindespindel 21, 22 und Gegengewinde 23, 24, die Gegengewinde 23, 24 sind als Gewindehülsen ausgebildet.

Die Gewindespindeln 21, 22 weisen an den äußeren Mantelflächen 34, 35 Außengewinde 18, 19 auf. Die Außengewinde 18, 19 der Gewindespindeln 21, 22 sind voneinander beabstandet. Die Längsachsen der Gewindespindeln 21, 22 verlaufen parallel zueinander und sind starr mit der oberen Platte 12 verbunden, d.h. die beiden Gewindespindeln 21, 22 und die obere Platte 12 mit den Dornen 14 bilden das obere Implantatteil 15, das einstückig ist.

Das untere Implantatteil 25 umfasst die untere Platte 16 mit den Dornen 14 sowie die beiden Gewindehülsen 23, 24, die drehbar in der Platte 16 gelagert sind.

An den inneren Mantelflächen 37, 38 der Gewindehülsen 23, 24 sind Gewinde 39, 40 vorgesehen, die die Spindelmuttern für die Außengewinde 18, 19 der Gewindespindeln 21, 22 bilden.

Eine jede Gewindehülse 23, 24 weist zudem an ihrer äußeren Mantelfläche ein Zahnrad 28, 29 auf. Die Zahnräder 28, 29 auf den Gewindehülsen 23, 24 bilden ein Zahnradpaar und koppeln die Rotationsbewegung der beiden Gewindehülsen.

Die Gewindehülsen 23, 24 sind in der unteren Platte 16 drehbar gelagert. Erfolgt eine Drehbewegung an einer Gewindehülse 23 (oder 24), so wird zum einen durch das Ineinandergreifen der Zahnradpaare 28, 29 die Drehbewegung auf die andere Gewindehülse 24 (oder 23) übertragen. Diese gekoppelten Zahnräder bewirken, dass der Spreizmechanismus mit einem doppelten Verstellmechanismus angetrieben wird.

Zum anderen bewirkt die Drehbewegung auf eine jede Gewindehülse 23, 24 jedoch auch, dass eine Verschiebebewegung der Gewindespindeln 21, 22 gegenüber den Gewindehülsen 23, 24 und damit eine Verschiebung der oberen Platte 12 gegenüber der unteren Platte 16 und damit ein Spreizen des Implantats erfolgt.

Auf diese Weise erfolgt das Aufspreizen des Implantats bei Drehung einer der beiden Gewindehülsen in einer gleichmäßigen Bewegung, und ein Verkanten der Gewinde wird vermieden.

Die Drehrichtungen der Gewindespindeln 21, 22 sind verschieden, ebenso die Drehrichtungen der Gewindehülsen 23, 24. Eine Drehbewegung auf ein Zahnrad bewirkt, dass sich das andere Zahnrad in die entgegengesetzte Richtung dreht, somit drehen sich auch die in der unteren Platte 16 drehbar, aber nicht höhenverstellbar gelagerten Gewindehülsen 23, 24 in entgegengesetzte Richtungen und infolge der unterschiedlichen Drehrichtungen der Gewindespindeln 21,22 wird die obere Platte 12 gegenüber der unteren Platte 16 angehoben.

Unterhalb des Zahnrads 29 befindet sich die Antriebswelle 30 (Tellerrad) eines Kegelradgetriebes, die sich von der Zahnung 27 des Zahnrades 29 radial nach außen erstreckt. Der äußere Durchmesser des Tellerrads 30 ist größer als der Durchmesser des Zahnrades 29. Somit kann der Kegelantrieb 61 des Bedieninstruments 60 an dem äußeren Umfang des Tellerrads 30 angreifen, ohne die Bewegung des Zahnrads 29 zu behindern.

Tellerrad 30, Gewindehülse 24 und Zahnrad 29 bilden eine starr miteinander verbundene Einheit. Die Zahnung 31 des Tellerrads 30 weist in Richtung der oberen Platte 12, d.h. in +z-Richtung, und verläuft etwa rechtwinklig zu der Zahnung 27 des Zahnrads 29, die sich in xy-Richtung erstreckt.

Durch eine Drehung des Tellerrads 30 wird gleichzeitig auch das Zahnrad 29 und damit - über die Kopplung der Zahnräder 28, 29 - auch das Zahnrad 28 und damit der beiden mit den Zahnrädern verbundenen Gewindehülsen 23, 24 gedreht, wodurch das Implantat gespreizt wird.

Parallel zu den Längsachsen (z-Richtung) der Gewindehülsen- und -spindeln 21, 23; 22, 24 ist ein starr mit der unteren Platte 16 verbundener, ebenfalls in Richtung der oberen Platte 12 weisender Abschnitt 43 mit einem Langloch 44 vorgesehen, in dem ein an der oberen Platte 12 befestigter Führungssteg 45, der an seinem Ende einen Anschlagbolzen 46 aufweist, geführt ist. Während des Spreizens gleitet der Führungssteg 45 in dem Langloch 44 nach oben, bis die maximale Spreizung erreicht ist und der am Ende des Führungsstegs 45 befindliche Anschlagbolzen 46 an dem oberen Ende des Langlochs 44 anliegt.

An der Gewindehülse 24 mit dem Tellerrad 30 ist an dem mit der unteren Platte 16 verbundenen Abschnitt 43 zudem ein Horizontalabschnitt 48 mit mehreren Gewindebohrungen 49 vorgesehen, in die ein korrespondierendes Schraubgewinde 66 am Bedieninstrument 60 bzw. Verschlussschrauben 78 eingeschraubt werden können. Aus Stabilitätsgründen ist der Horizontalabschnitt 48 mit den Gewindebohrungen 49 auch an der dem Abschnitt 43 mit dem Langloch 44 gegenüberliegenden Seite durch einen Steg 50 mit der unteren Platte 16 starr verbunden.

Untere Platte 16, Abschnitt 43, Horizontalabschnitt 48 mit den Bohrungen 49 und Steg 50 bilden eine starre Einheit.

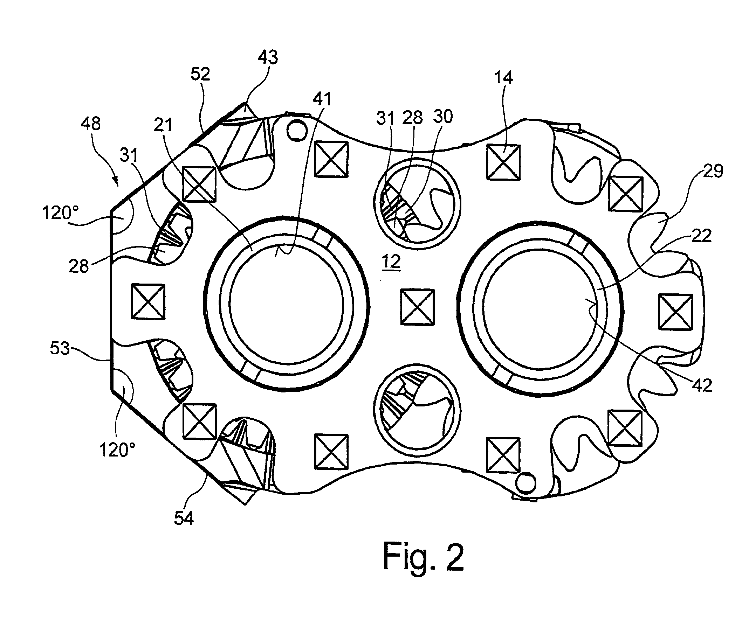

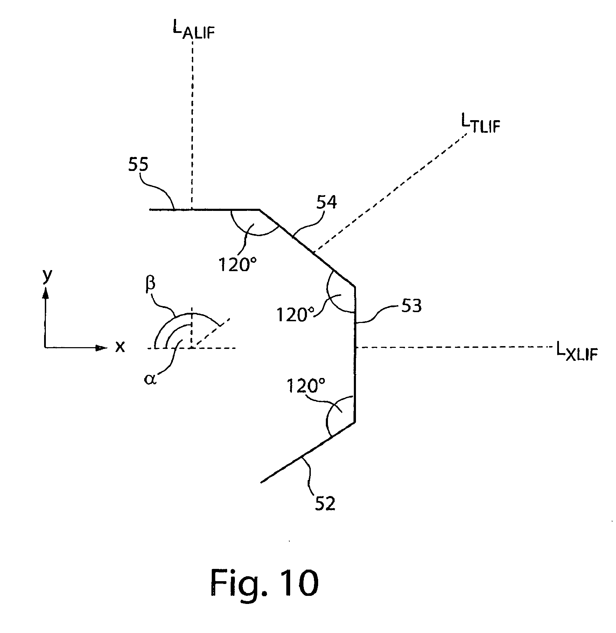

Insgesamt sind auf dem Horizontalabschnitt 48 sechs Gewindebohrungen 49 vorgesehen. In der in Figur 10 dargestellten weiteren Variante des Horizontalabschnitts 48 mit vier Teilabschnitten 52 bis 55 sind es insgesamt acht Bohrungen 49 (nicht dargestellt). Der Horizontalabschnitt 48 besteht aus drei Teilabschnitten 52, 53, 54, wobei sich an das Ende des ersten Abschnitts 52 in xy-Richtung der zweite Abschnitt 53 unter einem Winkel von 120° und an das gegenüberliegende Ende des zweiten Abschnitts 53 in x-y-Richtung der dritte Abschnitt 54, wiederum unter einem Winkel von 120°, anschließt (vgl. Fig. 2). Diese äußere Formgebung des Horizontalabschnitts 48 dient zur einfachen Positionierung des Bedieninstruments 60, wie anschließend beschrieben werden wird.

Die verschiedenen, unterschiedlich orientierten Gewindebohrungen 49 ermöglichen dem Arzt, das Implantat in der gewünschten Orientierung an dem Bedieninstrument 60 zu befestigen und in dieser Position von der gewünschten Seite einzubringen.

Jeweils zwei Gewindebohrungen 49 liegen auf einem der drei Teilabschnitte 52, 53, 54. Die Gewindebohrungen 49 dienen sowohl zur Befestigung des Schraubgewindes 66 an der Festziehwelle 72 des Bedieninstruments 60 als auch zur Fixierung der Spreizposition durch die Verschlussschraube 78. Der Abstand zweier Gewindebohrungen 49 auf einem Teilabschnitt entspricht dem Abstand zwischen den proximalen Enden der beiden Aufnahmerohre 70, 71 des Bedieninstruments 60, durch die das Implantat temporär mit dem Bedieninstrument 60 verschraubt und durch die die bleibende Fixierung der Spreizstellung mittels der Verschlussschraube 78 mit dem Schraubeninstrument 80 erfolgt.

Wird das Bedieninstrument 60 so mit dem Implantat verschraubt, dass es an dem Abschnitt 54 (oder 52) anliegt, so beträgt der Winkel zwischen der Längsachse des Bedieninstruments 60, die in Figur 10 als L_{TLIF} bezeichnet ist, und der Längsachse des Implantats ß = 125°. In dieser Befestigungsposition, in der die Längsachse des Implantats und die Längsachse des Bedieninstruments einen stumpfen Winkel einschließen, kann das Implantat auf einfache Weise von hinten in der TLIF-Technik (der Patient liegt in Bauchlage) implantiert werden, insbesondere aufgrund des stumpfen Winkels ß kann das Implantat um die Nervenwurzeln herum in die gewünschte Position zwischen den Wirbelkörpern geschoben werden, ohne diese zu beschädigen. Durch den Befestigungswinkel kann das Implantat zudem auch sicher nicht nur in irgendeine Position, sondern auch in die optimale Position gebracht werden, was mit den bislang bekannten, sich in Längsrichtung des Bedieninstruments erstreckenden Implantate rein geometrisch nicht möglich ist.

Wird das Bedieninstrument hingegen an dem Abschnitt 53 mit dem Implantat verschraubt, so greift das Bedieninstrument an der Schmalseite des Implantats an, d.h. die Längsachse des Implantats, die in Figur 10 als L_{XLIF} bezeichnet ist, und die Längsachse des Bedieninstruments verlaufen parallel zueinander. In dieser Befestigungsposition kann die Implantierung in der XLIF-Technik (Extreme Lateral Interbody Fusion) in Seitenlage des Patienten erfolgen, wie es insbesondere in Bezug auf den 4. Lendenwirbel erforderlich sein kann.

In einer weiteren Variante des Horizontalabschnitts 48, der in Figur 10 in Draufsicht dargestellt ist, schließt sich an den Abschnitt 54 unter einem Winkel von 120° noch ein weiterer Abschnitt 55 an, der, wie die anderen Abschnitte 52, 53 und 54, ebenfalls zwei Bohrungen 49 aufweist. Der Abschnitt 55 verläuft parallel der Längsachse des Implantats, d.h. in x-Richtung. Wird das Bedieninstrument an dem Abschnitt 55 befestigt, so stehen die Längsachse des Bedieninstruments 60, die in der Figur 10 mit L_{ALIF} bezeichnet ist, und die Längsachse des Implantats (x-Richtung) in einem Winkel a von 90° zueinander. Ein in dieser Position an dem Bedieninstrument 60 befestigtes Implantat eignet sich insbesondere zur ALIF- oder ALIF 30°-Implantation (Anterior Lumbar Interbody Fusion), in der der Patient in Rückenlage liegt und das Implantat insbesondere zum Ersatz des 5. Lendenwirbels vom Bauchraum aus eingebracht wird.

Um eine gute knöcherne Durchbauung zu gewährleisten, sind die Gewindespindeln 21, 22 hülsenförmig und innen hohl. Zudem weisen die Mantelflächen 34, 35 der Gewindespindeln 21, 22 und die Mantelflächen 26, 27 der Gewindehülsen 23, 24 Durchbrechungen 20 auf, die ebenfalls der knöchernen Durchbauung dienen.

Die Stirnflächen der Gewindehülsen 23, 24 und hülsenförmigen Gewindespindeln 21, 22 sind offen, d.h. nicht von der oberen Platte 12 bzw. unteren Platte 16 abgedeckt. Das Implantat weist somit zwei durchgängige, sich von der Oberseite 13 der oberen Platte 12 bis zur Unterseite 17 der unteren Platte 16 erstreckende rohrförmige Öffnungen auf, die mit Knochenmaterial befüllbar sind und durch die eine knöcherne Durchbauung von dem oben angrenzenden Wirbelkörper bis zum unten angrenzenden Wirbelkörper erreicht werden kann.

Die obere Platte 12 und die untere Platte 16 umfassen ebenfalls Öffnungen und Durchbrechungen 20, die der knöchernen Durchbauung dienen. Die Grundform der Platten 12, 16 ist oval und ähnlich einer "Acht" mit zueinander koplanaren Außenseiten 13, 17. Durch die Größe der Auflagefläche der Platten 12, 16 auf den Wirbelkörpern wird die Flächenbelastung der Wirbelkörper verringert und zudem die Gefahr eines Einsinkens des Implantats vermindert.

Die Dicke der oberen und unteren Platte 12, 16 ist relativ gering. Zudem sind die Hülsen 23, 24 und die Spindeln 21, 22 in den Platten 12, 16 befestigt und durchqueren diese und ein jedes Getriebe besteht genau aus einer Hülse 23, 24 und einer Spindel 21, 22. Hierdurch wird eine kompakte Bauweise mit maximalem Hub erreicht.

In einer Variante können die obere Platte 12 und/oder untere Platte 16 auch keilförmig ausgebildet sein, so dass die nach außen weisenden Flächen 13, 17 der oberen Platte 12 und unteren Platte 16 in einem spitzen Winkel gegeneinander geneigt sind. Hierdurch kann die Lordoseform erreicht werden.

Bei dem erfindungsgemäßen Implantat bildet die obere Platte 12 mit den Gewindespindeln 21, 22 und dem wahlweise vorhandenen Führungssteg 45 ein fest verbundenes Teil, die untere Platte 16 mit dem Abschnitt 43 und dem Horizontalabschnitt 48 bildet ebenfalls ein fest verbundenes Teil. Zudem ist eine jede Gewindehülse 23, 24 mit dem Zahnrad 28, 29 und ggf. der Antriebswelle 30 einteilig ausgebildet und die beiden Gewindehülsen 23, 24 sind in der unteren Platte 16 drehbar gelagert. Hierzu weist eine jede Gewindehülse 23, 24 an ihrer unteren Seite einen Ringbund auf, der mit der Hülse 23, 24 verpresst ist und der unterhalb der unteren Platte 16 an der Unterseite der unteren Platte 16 anliegt. Somit sind die Hülsen 23, 24 in der unteren Platte drehbar, aber nicht höhenverstellbar gelagert.

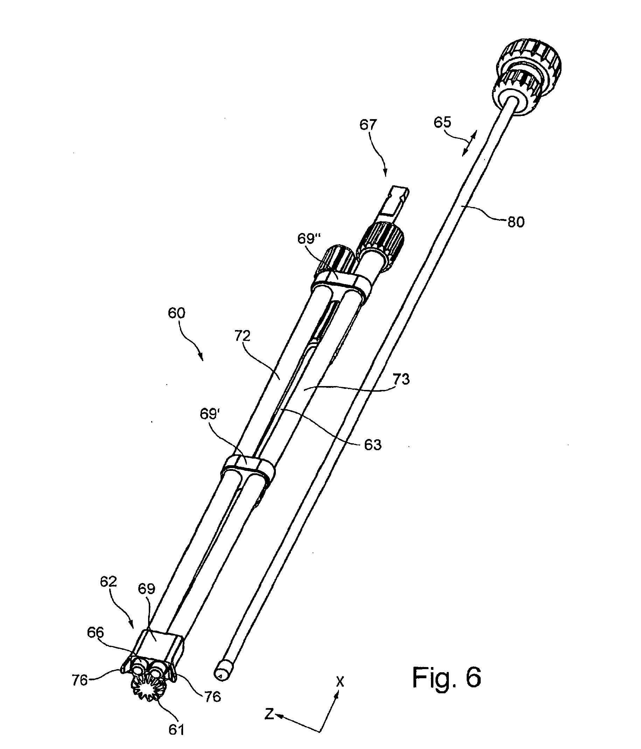

Das Implantat wird mit einem Instrument 60 bedient, das in den Figuren 6 ff. näher dargestellt ist. Das Instrument 60 dient sowohl zum Befestigen und Einbringen des Implantats als auch zum Aufspreizen und zur Fixierung der Spreizstellung.

Um das Implantat für die spätere Handhabung, d.h. Einbringen, Positionieren, Aufspreizen, sicher an dem Bedieninstrument 60 zu befestigen, umfasst das Instrument 60 zwei Festziehwellen 72, 73, die an ihrem proximalen Ende 62 jeweils ein Schraubgewinde 66, 74 umfassen. Mittels der Schraubgewinde 66, 74 kann das Instrument 60 mit dem Implantat an zwei benachbarten Gewindebohrungen 49, 49' auf einem der Abschnitte 52, 53 oder 54 an ihrem distalen Ende 67 durch Drehung fest verschraubt werden.

Zum Aufspreizen des Implantats umfasst das Instrument 60 eine Drehwelle 63, an deren proximalem Ende 62 ein Kegelantrieb 61 zum Antrieb des Kegelrads 30 vorgesehen ist. Die Betätigung des Kegelantriebs 61 erfolgt durch eine Drehbewegung am distalen Ende 67 der Welle 63.

Das Instrument 60 umfasst weiterhin zwei Rohre 70, 71, die dazu dienen, die Festziehwellen 72, 73 mit den Schraubgewinden 66, 74 aufzunehmen und zu führen. Die Rohre 70, 71 dienen auch dazu, einen Schraubendreher 80 aufzunehmen, um die Verschlussschraube 78 einzuschrauben, wenn die gewünschte Spreizstellung erreicht ist. Hierzu wird zunächst eine der beiden Festziehwellen 72, 73 mit Gewinde 66, 74 aus einem der Rohre 70, 71 entfernt und in das leere Aufnahmerohr 70, 71 das Schraubinstrument 80 hineingesteckt und die Verschlussschraube 78 in die Gewindebohrung 49 geschraubt.

Das Schraubinstrument 80 besteht aus einem Schraubstab 84 mit Rändelung und einem Verschlussschraubenrohr 83, in dessen proximalen Ende 62 die Verschlussschraube 78 hineingesteckt wird. Die Verschraubung erfolgt durch Drehung des Schraubstabs 84.

Durch die Verschlussschraube 78 wird die Gewindehülse 24 gegenüber der Gewindespindel 22 in der gewünschten Position verklemmt.

Durch die Verschraubung des Implantats mit den Schraubgewinden 66, 74 der Festziehwellen 72, 73 des Bedieninstruments 60 wird nicht nur eine starre Verbindung zwischen Bedieninstrument 60 und Implantat und damit ein präzises Einbringen erreicht, sondern gleichzeitig auch der Kegelantrieb 61 des Bedieninstruments 60 in Eingriff mit dem Tellerrad 30 gebracht, so dass nach erfolgter Verschraubung mit dem Implantat dann durch Drehen der Drehwelle 63 mit dem Kegelantrieb 61 das Implantat definiert aufgespreizt werden kann.

Die Längsachsen der beiden Aufnahmerohre 70, 71 und der Wellen 63, 72, 73 mit dem Kegelantrieb 61 bzw. den Schraubgewinden 66, 74 verlaufen parallel zueinander. Die beiden Rohre 70, 71 sind starr mit drei Aufnahmen 69, 69', 69" verbunden, die sich am proximalen 62, am distalen Ende 67 und auch mittig befinden. Die Drehwelle 63 mit dem Kegelantrieb 61 ist drehbar in den Aufnahmen 69, 69', 69" gelagert. Die Aufnahmen 69, 69', 69" und die beiden Rohre 70, 71 bilden eine starre Halterung für die Drehwelle 63 mit dem Kegelantrieb 61 und die Festziehwellen 72, 73 mit den Schraubgewinden 66, 74.

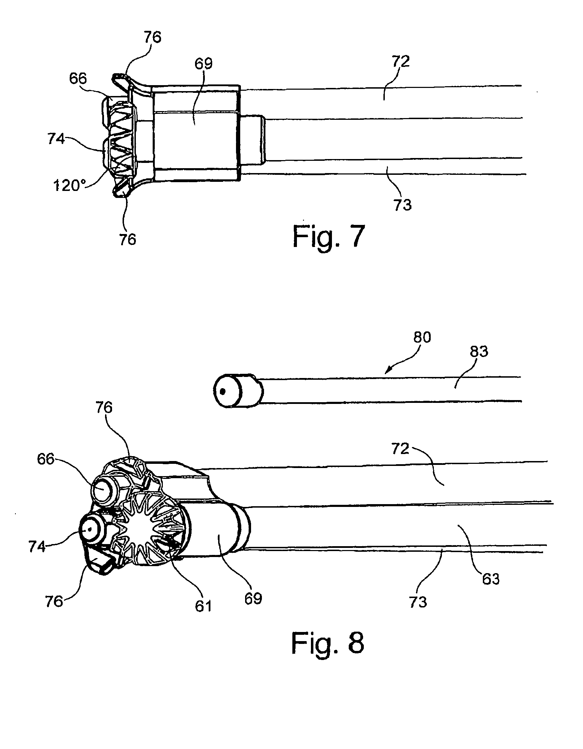

Zur besseren Positionierung des proximalen Endes 62 des Bedieninstruments 60 an dem Horizontalabschnitt 48 mit den Gewindebohrungen 49 weist die proximale Aufnahme 69 des Bedieninstruments 60 in proximale Richtung eine der Abwinkelung der Teilabschnitte 52, 53, 54 des Horizontalabschnitts 48 korrespondierende Form auf, nämlich zwei sich in proximale Richtung von der Aufnahme 69 unter einem Winkel von 120° seitlich erstreckende Positionierschenkel 76 (vgl. Figur 7, 8), die dem ebenfalls unter einem Winkel von 120° folgenden Verlauf des Horizontalabschnitts 48 entsprechen und so eine einfache und genaue Positionierung der Rohre 70, 71 vor den Gewindebohrungen 49, 49' zur Befestigung der Schraubgewinde 66, 74 der Festziehwellen 72, 73 und der Verschlussschrauben 78 mit dem Schraubinstrument 80 ermöglichen.

Die Drehwelle 63 mit dem Kegelantrieb 61 steht am distalen Ende 67 des Bedienelements 60 deutlich hervor, so dass der Arzt während der Operation nicht versehentlich statt des Aufspreizens die Verschraubung löst.

Durch die geringe Größe des Kegelantriebs 61 und die parallele Anordnung der Längsachsen der Wellen 63, 72, 73 und der Rohre 70, 71 kann das Bedieninstrument 60 in einer schlanken schmalen Form ausgebildet werden, was ein einfaches und präzises Einbringen von vorne, von hinten und auch seitlich erleichtert. Eine solche schlanke Form des Bedieninstruments 60 ermöglicht zudem eine gute Sicht auf den OP-Bereich.

Da der Antrieb des Implantats über das Tellerrad 30 mit dem Ritzel 61 des Bedieninstruments 60 erfolgt, greift das Bedieninstrument 60 an der xy-Ebene an. Die Längsachse 65 des Bedieninstruments 60 verläuft dabei etwa im rechten Winkel zu der Spreizrichtung (Längsachse) des Implantats.

## Patentansprüche

1. Spreizbares Implantat mit einer oberen Platte (12) und einer unteren Platte (16), die zur Verankerung an/in den Wirbelstützflächen dienen und wenigstens zwei Getrieben (21, 23; 22, 24) zum Aufspreizen des Implantats, die miteinander über Zahnräder (28, 29) gekoppelt sind,
wobei ein jedes Getriebe (21, 23; 22, 24) Gewindespindeln (21, 22) und Gewindehülsen (23, 24) umfasst, die Gewindespindeln (21, 22) starr mit der oberen Platte (12) verbunden sind und die Gewindehülsen (23, 24) drehbar in der unteren Platte (16) gelagert sind, und an dem einen Getriebe (22, 24) eine Antriebswelle (30) vorgesehen ist, die starr mit der einen Gewindehülse (24) verbunden ist,
**dadurch gekennzeichnet, dass** mittels der Antriebswelle (30) eine auf die Antriebswelle (61) eines Bedieninstruments (60) wirkende Kraft auf das Getriebe (22, 24) übertragen werden kann und das Implantat Mittel (49) umfasst, um das Bedieninstrument (60) an dem Implantat starr zu befestigen, zu verschrauben oder zu fixieren und dieses Mittel (49) mit der unteren Platte (16) starr verbunden sind

2. Implantat nach Anspruch 1 **dadurch gekennzeichnet, dass** die Antriebswelle (30) ein Tellerrad oder ein Kegelring ist.

3. Implantat nach einem der vorangegangenen Ansprüche, **dadurch gekennzeichnet, dass** in der unteren Platte die Gewindehülse (24) mit der Antriebswelle (30) gelagert ist.

4. Implantat nach einem der Ansprüche 1 bis 3, **dadurch gekennzeichnet, dass** der Abstand zwischen der Antriebswelle (30) des Implantats und den Mitteln (49) des Implantats, um das Bedieninstruments (60) an dem Implantat starr zu befestigen, zu verschrauben oder zu fixieren, nicht veränderbar ist.

5. Implantat nach einem der vorangegangenen Ansprüche, **dadurch gekennzeichnet, dass** die Mittel (49) des Implantats, um das Bedieninstrument (60) an dem Implantat starr zu befestigen, zu verschrauben oder zu fixieren Bohrungen, insbesondere Gewindebohrungen (49), sind.

6. Implantat nach einem der vorangegangenen Ansprüche, **dadurch gekennzeichnet, dass** die obere Platte (12) und die untere Platte (16) und/oder die Gewindespindeln (21, 22) und/oder die Gewindehülsen (23, 24) Durchbrechungen (31, 32, 33, 36) aufweisen und/oder die Stirnflächen der Gewindespindeln (21, 22) und der Gewindehülsen (23, 24) offen sind.

7. Implantat nach einem der vorangegangenen Ansprüche, **dadurch gekennzeichnet, dass** jede Gewindehülse (23, 24) mit dem Zahnrad (28, 29) und ggf. der Antriebswelle (30) einteilig ausgebildet ist.

8. Implantat nach einem der vorangegangenen Ansprüche, **dadurch gekennzeichnet, dass** eine jede Gewindehülse (23, 24) an ihrer unteren Seite einen Ringbund aufweist, der mit der Hülse (23, 24) fest verbunden ist und der an der Unterseite der unteren Platte (16) anliegt.

9. Implantat nach einem der vorangegangenen Ansprüche, **dadurch gekennzeichnet, dass** an der inneren Mantelfläche (37, 38) der Gewindehülsen (23, 24) Gewinde (39, 40) vorgesehen sind, die die Spindelmuttern für die Außengewinde (18, 19) der Gewindespindeln (21, 22) bilden.

10. Implantat nach einem der vorangegangenen Ansprüche, **dadurch gekennzeichnet, dass** die Drehrichtungen der Gewindehülsen (23, 24) unterschiedlich sind.

11. Implantat nach einem der vorangegangenen Ansprüche, **dadurch gekennzeichnet, dass** das Implantat mehrere unterschiedlich orientierte Teilabschnitte (52, 53, 54, 55) zur Befestigung des Bedieninstruments (60) aufweist.

12. Bedieninstrument (60) für ein Implantat nach einem der vorangegangenen Ansprüche, welches Bedieninstrument (60) eine Antriebswelle (61) zur Betätigung der Antriebswelle (30) des Implantats aufweist und Mittel (66, 74) zur Befestigung an den korrespondierenden Mitteln (49) des Implantats zur starren Befestigung, Verschraubung oder Fixierung des Bedieninstruments (60).

13. Bedieninstrument (60) nach Anspruch 12, **dadurch gekennzeichnet, dass** die Mittel zur Befestigung (66, 74) Schraubgewinde sind, insbesondere Außengewinde am proximalen Ende (62) einer Festziehwelle (72, 73).

14. Bedieninstrument (60) nach einem der Ansprüche 11 bis 13, **dadurch gekennzeichnet, dass** die Antriebswelle (61) ein Kegelantrieb (61) am proximalen Ende (62) einer Drehwelle (63) ist.

15. Bedieninstrument (60) nach einem der Ansprüche 11 bis 13, **dadurch gekennzeichnet, dass** das Bedieninstrument (60) eine Halterung mit Rohren (70, 71) und Aufnahmen (69, 69', 69") umfasst, wobei die Längsachsen der Rohre (70, 71) parallel zueinander verlaufen und die Rohre (70, 71) zur Aufnahme der Festziehwellen (72, 73) und/oder eines Schraubinstruments (80) dienen.

## Claims

1. Expandable implant with an upper plate (12) and a lower plate (16) which serve for anchoring on/in the vertebral support surfaces, and at least two gears (21, 23; 22, 24) for the expansion of the implant, which are coupled to each other via toothed wheels (28, 29), whereby each gear (21, 23; 22, 24) comprises threaded spindles (21, 22) and threaded sleeves (23, 24), the threaded spindles (21, 22) are connected rigidly to the upper plate (12), and the threaded sleeves (23, 24) are mounted rotatably in the lower plate (16), and on one gear (22, 24) a drive shaft (30) is provided, which is connected rigidly to the one threaded sleeve (24), **characterized in that** a force acting on the drive shaft (61) of an operating instrument (60) can be transferred to the gear (22, 24) by means of the drive shaft (30), and the implant comprises means (49) for rigidly securing, screwing or fixing the operating instrument (60) on the implant and these means (49) being rigidly connected to the lower plate (16) .

2. Implant according to Claim 1, **characterized in that** the drive shaft (30) is a ring gear or a taper ring.

3. Implant according to one of the preceding claims, **characterized in that** the threaded sleeve (24) with the drive shaft (30) is mounted in the lower plate (16).

4. Implant according to one of Claims 1 to 3, **characterized in that** the distance between the drive shaft (30) of the implant and the means (49) of the implant for rigidly securing, screwing or fixing the operating instrument (60) is not modifiable.

5. Implant according to one of the preceding claims, **characterized in that** the means (49) of the implant for rigidly securing, screwing or fixing the operating instrument (60) to the implant are bores, in particular threaded bores (49).

6. Implant according to one of the preceding claims, **characterized in that** the upper plate (12) and the lower plate (16) and/or the threaded spindles (21, 22) and/or the threaded sleeves (23, 24) have apertures (31, 32, 33, 36) and/or the front faces of the threaded spindles (21, 22) and of the threaded sleeves (23, 24) are open.

7. Implant according to one of the preceding claims, **characterized in that** each threaded sleeve (23, 24) is formed in one piece with the toothed wheel (28, 29) and, if appropriate, the drive shaft (30) .

8. Implant according to one of the preceding claims, **characterized in that** each threaded sleeve (23, 24) has, on its underside, an annular collar which is fixedly connected to the sleeve (23, 24) and which bears on the underside of the lower plate (16) .

9. Implant according to one of the preceding claims, **characterized in that** the inner curved surface (37, 38) of the threaded sleeves (23, 24) is provided with threads (39, 40), which form the spindle nuts for the outer threads (18, 19) of the threaded spindles (21, 22).

10. Implant according to one of the preceding claims, **characterized in that** the directions of rotation of the threaded sleeves (23, 24) are different.

11. Implant according to one of the preceding claims, **characterized in that** the implant has a plurality of differently oriented sub-portions (52, 53, 54, 55) for securing the operating instrument (60).

12. Operating instrument (60) for an implant according to one of the preceding claims, which operating instrument (60) has a drive shaft (61) for actuation of the drive shaft (30) of the implant, and means (66, 74) for securing to the corresponding means (49) of the implant for rigidly securing, screwing or fixing the operation instrument (60).

13. Operating instrument (60) according to Claim 12, **characterized in that** the means (66, 74) are screw threads, in particular outer threads at the proximal end (62) of a tightening shaft (72, 73).

14. Operating instrument (60) according to either of Claims 12 and 13, **characterized in that** the drive shaft (61) is a bevel wheel gear (61) at the proximal end (62) of a rotation shaft (63).

15. Operating instrument (60) according to one of Claims 12 to 14, **characterized in that** the operating instrument (60) comprises a retainer with tubes (70, 71) and holders (69, 69', 69"), wherein the longitudinal axes of the tubes (70, 71) extend parallel to each other, and the tubes (70, 71) serve to receive the tightening shafts (72, 73) and/or a screw instrument (80).

## Revendications

1. Implant extensible avec une plaque supérieure (12) et une plaque inférieure (16) lesquelles servent à l'ancrage sur/dans les surfaces d'appui de vertèbre et avec au moins deux engrenages (21, 23; 22, 24) destinés à écarter l'implant, lesquels sont couplés entre eux par des roues dentées (28, 29), chacun desdits engrenages (21, 23; 22, 24) comprenant des tiges filetées (21, 22) et des manchons filetés (23, 24), les tiges filetées (21, 22) étant reliées de manière rigide à la plaque supérieure (12) et les manchons filetés (23, 24) étant logés dans la plaque inférieure (16) de manière à pouvoir tourner, sur ledit engrenage (22, 24) étant prévu un arbre d'entraînement (30) qui est relié de manière rigide audit manchon fileté (24),
**caractérisé en ce qu'**au moyen de l'arbre d'entraînement (30), une force agissant sur l'arbre d'entraînement (61) d'un instrument de manipulation (60) peut être transmise à l'engrenage (22, 24) et **en ce que** l'implant comprend des moyens (49) en vue de monter, visser ou fixer de manière rigide l'instrument de manipulation (60) à l'implant et que lesdits moyens (49) sont reliés de manière rigide à la plaque inférieure (16).

2. Implant selon la revendication 1, **caractérisé en ce que** l'arbre entraînement (30) est une couronne ou un anneau conique.

3. Implant selon l'une quelconque des revendications précédentes, **caractérisé en ce que** le manchon fileté (24) est logé, avec l'arbre entraînement (30), dans la plaque inférieure.

4. Implant selon l'une quelconque des revendications 1 à 3, **caractérisé en ce que** l'écart entre l'arbre d'entraînement (30) de l'implant et les moyens (49) de l'implant permettant de monter, visser ou fixer de manière rigide l'instrument de manipulation (60) à l'implant n'est pas modifiable.

5. Implant selon l'une quelconque des revendications précédentes, **caractérisé en ce que** les moyens (49) de l'implant permettant de monter, visser ou fixer de manière rigide l'instrument de manipulation (60) à l'implant sont des trous, en particulier des trous filetés (49).

6. Implant selon l'une quelconque des revendications précédentes, **caractérisé en ce que** la plaque supérieure (12) et la plaque inférieure (16) et/ou les tiges filetées (21, 22) et/ou les manchons filetés (23, 24) présentent des ajours (31, 32, 33, 36) et/ou que les faces frontales des tiges filetées (21, 22) et des manchons filetés (23, 24) sont ouvertes.

7. Implant selon l'une quelconque des revendications précédentes, **caractérisé en ce que** chaque manchon fileté (23, 24) est réalisé en une seule pièce avec la roue dentée (28, 29) et, le cas échéant, avec l'arbre d'entraînement (30).

8. Implant selon l'une quelconque des revendications précédentes, **caractérisé en ce que** chaque manchon fileté (23, 24) présente, sur son côté inférieur, un collet annulaire qui est relié de manière fixe au manchon (23, 24) et qui repose contre la face inférieure de la plaque inférieure (16).

9. Implant selon l'une quelconque des revendications précédentes, **caractérisé en ce que** sur la surface latérale intérieure (37, 38) des manchons filetés (23, 24) sont prévus des filets (39, 40) qui forment les écrous de tige pour les filets extérieurs (18, 19) des tiges filetées (21, 22).

10. Implant selon l'une quelconque des revendications précédentes, **caractérisé en ce que** les sens de rotation des manchons filetés (23, 24) sont différents.

11. Implant selon l'une quelconque des revendications précédentes, **caractérisé en ce que** l'implant présente plusieurs sections (52, 53, 54, 55) d'orientation différente en vue de la fixation de l'instrument de manipulation (60).

12. Instrument de manipulation (60) pour un implant selon l'une quelconque des revendications précédentes, lequel instrument de manipulation (60) présente un arbre d'entraînement (61) destiné à actionner l'arbre entraînement (30) de l'implant ainsi que des moyens (66, 74) destinés à être fixés sur les moyens correspondants (49) de l'implant en vue du montage, vissage ou de la fixation rigide de l'instrument de manipulation (60).

13. Instrument de manipulation (60) selon la revendication 12, **caractérisé en ce que** les moyens de fixation (66, 74) sont des filets de vissage, en particulier des filets extérieurs situés à l'extrémité proximale (62) d'un arbre de serrage (72, 73).

14. Instrument de manipulation (60) selon l'une quelconque des revendications 11 à 13, **caractérisé en ce que** l'arbre d'entraînement (61) est un arbre conique (61) situé à l'extrémité proximale (62) d'un arbre de rotation (63).

15. Instrument de manipulation (60) selon l'une quelconque des revendications 11 à 13, **caractérisé en ce que** l'instrument de manipulation (60) comprend une fixation pourvue de tubes (70, 71) et de logements (69, 69', 69"), les axes longitudinaux des tubes (70, 71) s'étendant parallèlement entre eux et les tubes (70, 71) servant à recevoir les arbres de serrage (72, 73) et/ou un instrument de vissage (80).
